(19) Europäisches Patentamt
European Patent Office
Office européen des brevets

(11) Veröffentlichungsnummer: **0 161 478**
**A2**

# (12) EUROPÄISCHE PATENTANMELDUNG

(21) Anmeldenummer: **85104246.5**

(22) Anmeldetag: **11.04.85**

(51) Int. Cl.[4]: **C 07 D 401/04**, C 07 D 401/14, A 61 K 31/505

(30) Priorität: **11.04.84 HU 139484**

(43) Veröffentlichungstag der Anmeldung: **21.11.85 Patentblatt 85/47**

(84) Benannte Vertragsstaaten: **AT BE CH DE FR GB IT LI NL SE**

(71) Anmelder: **RICHTER GEDEON VEGYESZETI GYAR R.T., Gyömröi ut 19-21, H-1475 Budapest X (HU)**

(72) Erfinder: **Bernáth, Gábor, Dr., Mérei u. 8, H-6722 Szeged (HU)**
Erfinder: **Kóbor, Jenö, Dr., Jósika u. 7, H-6722 Szeged (HU)**
Erfinder: **Lázár, János, Dr., Szamos u. 18/A, H-6723 Szeged (HU)**
Erfinder: **Motika, Gábor, Dr., Vajda u. 18/A, H-6723 Szeged (HU)**
Erfinder: **Ezer, Elemér, Lupényu 6-8, H-1026 Budapest (HU)**
Erfinder: **Hajós, György, Dr., Gábor Aron u. 59, H-1026 Budapest (HU)**
Erfinder: **Pálosi, Eva, Dr., Vend u. 21, H-1025 Budapest (HU)**
Erfinder: **Dénes, László, Dr., Szél u. 19, H-1035 Budapest (HU)**
Erfinder: **Szporny, László, Dr., Szaboicska M. u. 7, H-1114 Budapest (HU)**

(74) Vertreter: **Beszédes, Stephan G. Dr., Münchener Strasse 80a Postfach 1168, D-8060 Dachau (DE)**

(54) **1-2'-(substituierte)-5', 6', 7', 8'-Tetra-(hydro)-4'-(oxo)-chinazolin -6'-yli-3,4-di-hydrol-6,7-di-substituiertel-isochinolinderivate, Verfahren zu ihrer Herstellung und diese enthaltende Arzneimittel.**

(57) Gegenstand der Anmeldung sind 1-[2'-(substituierte)-5',6',7',8'-Tetra-(hydro)-4'-(oxo)-chinazolin-6'-yl]-3,4-di-[hydro]-6,7-di-[substituierte]-isochinolinderivate der allgemeinen Formel

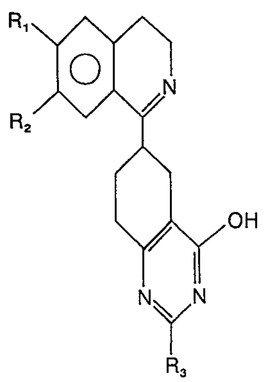

beziehungsweise I,

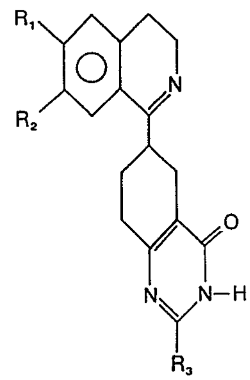

worin

$R_1$ und $R_2$ unabhängig voneinander für Hydroxygruppen beziehungsweise Alkoxyreste mit 1 bis 6 Kohlenstoffatom(en) stehen und

$R_3$ einen Alkylrest mit 1 bis 6 Kohlenstoffatome(en), einen, gegebenenfalls substituierten, Phenylrest, einen, gegebenenfalls substituierten, Alkylphenylrest mit 1 bis 4 Kohlenstoffatom(en) im Alkylteil oder einen, gegebenenfalls substituierten, heterocyclischen Rest bedeutet,

(Fortsetzung nächste Seite)

EP 0 161 478 A2

sowie ihre Säureadditionssalze und quaternären Salze und ein Verfahren zu ihrer Herstellung und diese Verbindungen enthaltende Arzneimittel, insbesondere mit krampflösenden, schmerz- und fiebersenkenden und bei akuter Alkoholvergiftung schützender Wirkungen.

Beschreibung

Die Erfindung betrifft neue 6,7-di-(substituierte) 3,4-Di-(hydro)-isochinolinderivate einschließlich ihrer Säureadditionssalze und quaternären Salze, ein Verfahren zu ihrer Herstellung und diese Verbindungen enthaltende Arzneimittel, insbesondere mit krampflösenden, schmerz- und fiebersenkenden und bei akuter Alkoholvergiftung schützenden Wirkungen.

Im Schrifttum wurden schon zahlreiche pharmazeutisch wirksame Verbindungen mit Isochinolingerüst beschrieben (siehe zum Beispiel: Ehrhart-Ruschig, Arzneimittel; S. Ebel: Synthetische Arzneimittel, Verlag Chemie). Es sind auch pharmazeutisch wirksame Chinazolinonderivate bekannt (in der Serie von Armarego, W. L. F.: Quinazolines - Fused Pyrimidines, Teil I, Interscience Publishers, New York, 1967). Nicht bekannt sind jedoch Verbindungen, welche die Kombination von diesen 2 Ringsystemen aufweisen.

Der Erfindung liegt die Aufgabe zugrunde, neue 6,7-di-(substituierte) 3,4-Di-(hydro)-isochinolinderivate mit überlegenen pharmakologischen, insbesondere krampflösenden, schmerz- und fiebersenkenden und bei akuter Alkoholvergiftung schützenden, Wirkungen, ein Verfahren zu ihrer Herstellung und diese enthaltende Arzneimittel zu schaffen.

Das Obige wurde überraschenderweise durch die Erfindung erreicht.

Gegenstand der Erfindung sind 1-[2'-(substituierte)-5',6',7',8'-Tetra-(hydro)-4'-(oxo)-chinazolin-6'-yl]-3,4-di-[hydro]-6,7-di-[substituierte]-isochinolinderivate [beziehungsweise als 4'-(Hydroxy)-Tautomere] beziehungsweise 1-[2'-(substituierte)-3',4',5',6',7',8'-Hexa-(hydro)-4'-(oxo)-chinazolin-6'-yl]-3,4-di-[hydro]-6,7-di-[substituierte]-isochinolinderivate der allgemeinen Formel

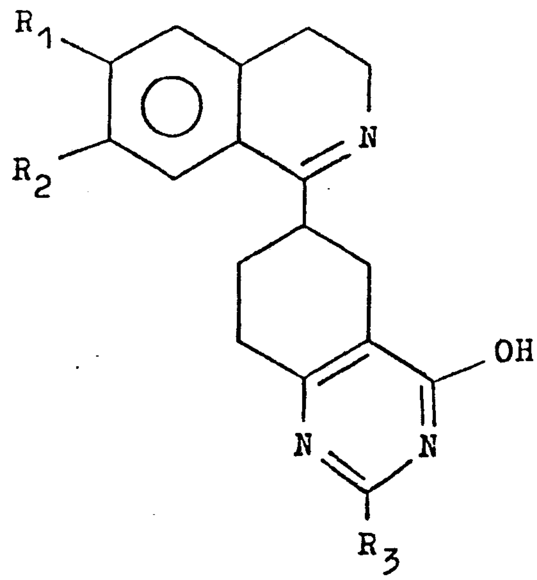

beziehungsweise I,

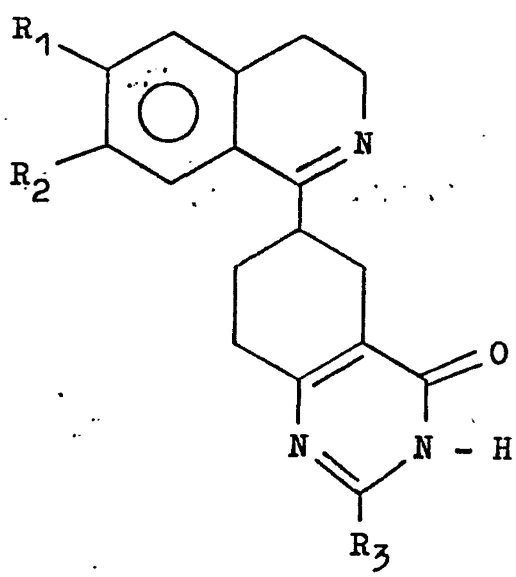

worin

$R_1$ und $R_2$ unabhängig voneinander für Hydroxygruppen beziehungsweise Alkoxyreste mit 1 bis 6 Kohlenstoffatom(en) stehen und

$R_3$ einen Alkylrest mit 1 bis 6 Kohlenstoffatom(en), einen, gegebenenfalls substituierten, Phenylrest, einen, gegebenenfalls substituierten, Alkylphenylrest mit 1 bis 4 Kohlenstoffatom(en) im Alkylteil oder einen, gegebenenfalls substituierten, heterocyclischen Rest bedeutet,

sowie ihre Säureadditionssalze und quaternären Salze.

Die Alkylreste, sowohl sofern sie an sich als auch sofern sie als Teile von anderen Resten vorliegen, sind gesättigt und können geradkettig oder verzweigt mit je nach der für die Kohlenstoffatomzahl oben jeweils angegebenen Begrenzung sein. Beispiele sind Methyl-, Äthyl-, n-Propyl-, Isopropyl-, n-Butyl-, sek.-Butyl-, tert.-Butyl-, n-Pentyl-, Isopentyl-, n-Hexyl- und Isohexylreste.

Vorzugsweise ist beziehungsweise sind der beziehungsweise die Alkoxyrest(e) und/oder Alkylrest(e), für welche[n] $R_1$ und/oder $R_2$ und/oder $R_3$ stehen kann beziehungsweise können, [ein] solche[r] mit 1 bis 4, insbesondere 1 oder 2, Kohlenstoffatom(en).

Es ist auch bevorzugt, daß der Alkylphenylrest, für welchen $R_3$ stehen kann, ein solcher mit 1 oder 2, insbesondere 1 [Benzylrest], Kohlenstoffatom(en) im Alkylteil ist.

Ferner ist es bevorzugt, daß der beziehungsweise die Substituent(en), durch welche[n] der Phenylrest oder Alkyl-

phenylrest, für den $R_3$ stehen kann, substituiert sein kann, 1 oder mehr Halogenatom(e) wie Chlor-, Fluor-, Brom- und/oder Jodatom(e), insbesondere Chloratom(e), Alkoxyrest(e) mit 1 bis 6, insbesondere 1 bis 4, ganz besonders 1 oder 2, vor allem 1 [Methoxyrest(e)], Kohlenstoffatom(en), Alkylrest(e) mit 1 bis 6, insbesondere 1 bis 4, ganz besonders 1 oder 2, vor allem 1 [Methylrest(e)], Kohlenstoffatom(en) und/oder Trihalogenmethylrest(e), insbesondere Trifluormethylrest(e), ist beziehungsweise sind. Falls mehr als 1 Substituent vorliegt, können also diese gleich oder verschieden sein. Sofern die Phenyl- beziehungsweise Alkylphenylreste, für welche $R_3$ stehen kann, substituiert sind, ist ihre Substitution bevorzugt in der beziehungsweise in den 2- und/oder 3-Stellung(en).

Der heterocyclische Rest, für den $R_3$ stehen kann, kann ein gesättigter oder ungesättigter carbocyclischer Rest, der durch 1 oder mehr gleiche oder verschiedene Heteroatom(e) unterbrochen ist, sein. Dabei ist es bevorzugt, daß der heterocyclische Rest, für den $R_3$ stehen kann, 5 bis 7-gliedrig, insbesondere 5- oder 6-gliedrig, ganz besonders 6-gliedrig, ist und als Heteroatom(e) 1 oder mehr Stickstoff-, Sauerstoff- und/oder Schwefelatom(e) aufweist, vor allem ein Pyridyl-, wie Pyrid-3-yl-, Piperidinyl-, wie Piperidin-1-yl-, Morpholinyl-, wie Morpholin-4-yl-, Piperazinyl-, wie Piperazin-1-yl-, 1,2,5,6-Tetrahydropyridyl-, wie 1,2,5,6-Tetrahydropyrid-1-yl-, oder Pyrrolidinylrest, wie Pyrrolidin-1-ylrest, ist. Als Heteroatom(e) ist beziehungsweise sind [ein] Stickstoff- und/oder Sauerstoffatom(e) besonders bevorzugt.

Weiterhin ist es bevorzugt, daß der beziehungsweise die Substituent(en), durch welche[n] der heterocyclische Rest, für den $R_3$ stehen kann, substituiert sein kann, 1 oder mehr Alkylrest(e) mit 1 bis 4, insbesondere 1 oder 2, ganz besonders 1, Kohlenstoffatom(en), gegebenenfalls durch 1 oder mehr Halogenatom(e), wie Chlor-, Fluor-, Brom- und/oder

Jodatom(e), insbesondere Chloratom(e), substituierte[r], Phenylrest(e), Hydroxygruppe(n), Carbamoylrest(e) und/oder weitere[r], vorteilhaft 5- bis 7-gliedrige[r], insbesondere 5- oder 6-gliedrige[r], ganz besonders 6-gliedrige[r], und als Heteroatom(e) 1 oder mehr Stickstoff-, Sauerstoff- und/oder Schwefelatom(e) aufweisende[r], heterocyclische[r] Rest(e), vor allem [ein] Pyridyl-, wie Pyrid-3-yl-, Piperidinyl-, wie Piperidin-1-yl-, Morpholinyl-, wie Morpholin-4-yl-, Piperazinyl-, wie Piperazin-1-yl-, 1,2,5,6-Tetrahydropyridyl-, wie 1,2,5,6-Tetrahydropyrid-1-yl-, oder Pyrrolidinylrest, wie Pyrrolidin-1-ylrest, ist beziehungsweise sind. Falls mehr als 1 Substituent vorliegt, können also diese gleich oder verschieden sein. Als Heteroatom(e) im beziehungsweise in den vorstehenden heterocyclischen Rest(en) ist beziehungsweise sind wiederum [ein] Stickstoff- und/oder Sauerstoffatom(e) besonders bevorzugt. Ganz besonders bevorzugt ist beziehungsweise sind von ihnen [ein] Piperidinylrest(e). In den Piperidinyl-, Piperazinyl- beziehungsweise Pyridylresten, für welche $R_3$ stehen kann, ist beziehungsweise sind der beziehungsweise die gegebenenfalls vorliegende(n) Substituent(en) bevorzugt in der 4-Stellung. Sofern der beziehungsweise die Phenylrest(e), durch welche[n] der heterocyclische Rest, für den $R_3$ stehen kann, substituiert ist beziehungsweise sind, ist ihre Substitution bevorzugt in der beziehungsweise den 2- und/oder 3-Stellung(en).

Ganz besonders bevorzugte erfindungsgemäße Verbindungen sind 1-[2'-(Piperidin-1''-yl)-5',6',7',8'-tetra-(hydro)-4'-(oxo)-chinazolin-6'-yl]-3,4-di-[hydro]-6,7-di-[methoxy]-isochinolin, 1-[2'-(4''-{p-⟨Chlor⟩-phenyl}-4''-{hydroxy}-piperidin-1''-yl)-5',6',7',8'-tetra-(hydro)-4'-(oxo)-chinazolin-6'-yl]-3,4-di-[hydro]-6,7-di-[methoxy]-isochinolin, 1-[2'-(4"-{Piperidin-1'''-yl}-4"-{carbamoyl}-piperidin-1"-yl)-5',6',7',8'-tetra-(hydro)-4'-(oxo)-chinazolin-6'-yl]-3,4-di-[hydro]-6,7-di-[methoxy]-isochinolin, 1-[2'-(3",4",5"-Tri-{methoxy}-phenyl)-

0161478

-5',6',7',8'-tetra-(hydro)-4'-(oxo)-chinazolin-6'-yl]-3,4-di--[hydro]-6,7-di-[methoxy]-isochinolin, 1-[2'-(1",2",5",6"--Tetra{hydro}-4"-{phenyl}-pyrid-1"-yl)-5',6',7',8'--tetra-(hydro)-4'-(oxo)-chinazolin-6'-yl]-3,4-di-[hydro]--6,7-di-[methoxy]-isochinolin, 1-[2'-(4"-{2'''-⟨Chlor⟩--phenyl}-piperazin-1"-yl)-5',6',7',8'-tetra-(hydro)--4'-(oxo)-chinazolin-6'-yl]-3,4-di-[hydro]-6,7-di-[methoxy]--isochinolin, 1-[2'-(3"-⟨Methyl⟩-phenyl)-5',6',7',8'--tetra-(hydro)-4'-(oxo)-chinazolin-6'-yl]-3,4-di-[hydro]-6,7--di-[methoxy]-isochinolin, 1-[2'-(pyrid-3"-yl)-5',6',7',8'--tetra-(hydro)-4'-(oxo)-chinazolin-6'-yl]-3,4-di--[hydro]-6,7-di-[methoxy]-isochinolin und 1-[2'-(Pyrrolidin--1"-yl)-5',6',7',8'-tetra-(hydro)-4'-(oxo)-chinazolin--6'-yl]-3,4-di-[hydro]-6,7-di-[methoxy]-isochinolin [beziehungsweise jeweils als 4'-(Hydroxy)-Tautomer] sowie ihre Säureadditionssalze und quaternären Salze. Auch in den obigen Verbindungen ist der Ausdruck "-5',6',7',8'-tetra-(hydro)-" als wahlweise auch "-3',4',5',6',7',8'-hexa-(hydro)-" umfassend zu verstehen, da die erfindungsgemäßen Verbindungen tautomer (Keto/Enol-Tautomerie) sind.

Gegenstand der Erfindung ist auch ein Verfahren zur Herstellung der erfindungsgemäßen Verbindungen, welches dadurch gekennzeichnet ist, daß 1-[3'-(Carboxy)-4'-(oxo)-cyclohex-1'--yl]-3,4-di-[hydro]-6,7-di-[substituierte]-isochinolinderivate beziehungsweise 1-[3'-(Carboxy)-4'-(imino)-cyclohex-1'--yl]-3,4-di-[hydro]-6,7-di-[substituierte]-isochinolinderivate der allgemeinen Formel

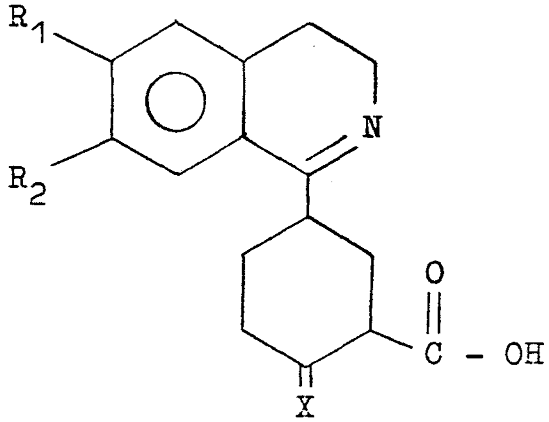

II ,

worin

$R_1$ und $R_2$ die oben angegebenen Bedeutungen haben und

X für ein Sauerstoffatom oder eine Iminogruppe (Gruppe der Formel = N - H) steht,

oder reaktionsfähige Derivate derselben mit Amidinen der allgemeinen Formel

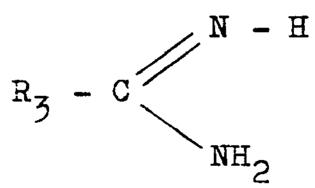

III ,

worin $R_3$ die oben angegebenen Bedeutungen hat, oder mit Salzen derselben mit anorganischen oder organischen Säuren in einem basischen Medium kondensiert werden,

worauf

gegebenenfalls in den erhaltenen 1-[2'-(substituierte)-5',6',7',8'-Tetra-(hydro)-4'-(oxo)-chinazolin-6'-yl]-3,4-di-[hydro]-6,7-di-[substituierte]-isochinolinderivaten der allgemeinen Formel I der beziehungsweise die Substituent(en), für welche[n] $R_1$ und/oder $R_2$ und/oder $R_3$ steht beziehungsweise stehen, zu [einem] anderen Substituenten, welche[n] $R_1$ und/oder $R_2$ und/oder $R_3$ bedeuten kann beziehungsweise können, umgesetzt wird beziehungsweise werden

und/oder

gegebenenfalls die erhaltenen 1-[2'-(substituierte)-5',6',7',8'-Tetra-(hydro)-4'-(oxo)-chinazolin-6'-yl]-3,4-di-[hydro]-6,7-di-[substituierte]-isochinolinderivate der allgemeinen Formel I in Säureadditionssalze oder quaternäre Salze überführt werden und/oder gegebenenfalls die erhaltenen Säureadditionssalze der 1-[2'-(substituierte)-5'6',7',8'-Tetra-(hydro)-4'-(oxo)-chinazolin-6'-yl]-3,4-di-[hydro]-6,7-di-[substituierte]-isochinolinderivate der allgemeinen Formel I in die freien 1-[2'-(substituierte)-5',6',7',8'-Tetra-(hydro)-4'-(oxo)-chinazolin-6'-yl]-3,4-di-[hydro]-6,7-di-[substituierte]-isochinolinderivate der allgemeinen Formel I oder in andere Säureadditionssalze oder quaternäre Salze überführt werden.

Die zur Herstellung der Verbindungen der allgemeinen Formel I als Ausgangsstoffe verwendeten Ketoester der allgemeinen Formel II, worin $R_1$ , $R_2$ und X wie oben festgelegt sind - können nach der gleichzeitig eingereichten der ungarischen Patentanmeldung vom 11. April 1984 mit dem Aktenzeichen Nr. 1393/84 entsprechenden europäischen Patentanmeldung derselben Anmelderin zum Beispiel durch den Ringschluß aus den entsprechenden Diestern hergestellt werden. Die in diesem Verfahren angewendeten Diester sind entweder bekannte Verbindungen oder können auf bekannte Weise hergestellt werden, z. B. aus in der Stellung 6,7 nichtsubstituierten bekannten Diestern oder aus den entsprechenden disubstituierten 1-Methyl-3,4-dihydro-isochinolin-Derivaten, die bekannte Verbindungen sind.

Als reaktionsfähige Derivate der Säuren der allgemeinen Formel II können z. B. ihre Ester oder Säureamide, vorzugsweise die letzteren, angewendet werden.

Die Verbindungen der allgemeinen Formel III - worin $R_3$ wie oben definiert ist - sind ebenfalls



BAD ORIGINAL

bekannt, und können durch in der organischen Chemie allgemein bekannte Verfahren, so zum Beispiel durch das Umsetzen von entsprechenden Iminoäthern oder deren Salzen und Ammoniak, hergestellt werden.

Die in einem basischen Medium durchgeführte Kondensation von Säuren der allgemeinen Formel II und Verbindungen der allgemeinen Formel III bzw. der mit anorganischen oder organischen Säuren gebildeten Salze der letzteren erfolgt selbst bei Raumtemperatur mit einer entsprechenden Geschwindigkeit, notwendigenfalls kann die Reaktion jedoch durch Erhönen der Temperatur beschleunigt werden.

Für die alkalische Kondensation kommen als Katalysatoren Alkalimetallhydroxide, wie Natrium- oder Kaliumhydroxid, Alkalimetallcarbonate, wie Natrium- oder Kalium-carbonat, oder Alkalimetallalkoholate, wie Natrium- oder Kaliumalkoholat in Frage.

Das Umsetzen kann in einem wäßrigen oder ein organisches Lösungsmittel enthaltender Medium erfolgen. Als organisches Lösungsmittel werden vorteilhaft Alkoholate, wie Methanol, Äthanol, aromatische organische Lösungsmittel, wie Benzol, Äther, zum Beispiel Tetrahydrofuran, eingesetzt.

In den Verbindungen der allgemeinen Formel I können die Substituenten $R_1$ und/oder $R_2$ und/oder $R_3$ erwünschtenfalls zu anderen, unter die früher angegebene Definition fallenden Substituenten $R_1$, $R_2$ beziehungsweise $R_3$ umgesetzt werden. So können aus den Verbindungen, die an der Stelle von $R_1$ und/oder $R_2$ eine Alkoxygruppe mit 1-6 Kohlenstoffatomen enthalten, durch Desalkylierung die entsprechenden Verbindungen hergestellt werden, die an der Stelle von $R_1$ und/oder $R_2$ eine Hydroxylgruppe enthalten. Die Desalkylierung kann auf bekannte Weise, zum Beispiel durch Erwärmen mit Brom- oder Jodwasserstoffsäure oder mit Hilfe von wasserfreiem Aluminiumchlorid erfolgen. Umgekehrt können die an der Stelle von $R_1$

- 11 -

BAD ORIGINAL

und/oder $R_2$ eine Hydroxylgruppe enthaltenden Verbindungen auf bekannte Weise zu den entsprechenden Alkoxyderivaten umgesetzt werden. Das Methylieren wird vorteilhaft mit Diazomethan oder Dimethylsulfat durchgeführt, während die Äther mit höherer Kohlenstoffatomanzahl zum Beispiel durch die Synthese nach Williamson, unter Verwendung von Alkyljodiden können hergestellt werden.

Aus den Verbindungen der allgemeinen Formel I können durch Umsetzen mit geeigneten Säuren Säureadditionssalze gebildet werden. Die Salzbildung wird in einem organischen Lösungsmittel, zum Beispiel einem aliphatischen Alkohol mit 1-6 Kohlenstoffatomen vorgenommen, indem man die Verbindung der allgemeinen Formel I suspendiert oder auflöst und zu der Lösung so lange die entsprechende Säure oder deren mit einem Lösungsmittel bereitete Lösung gibt, bis das Gemisch schwach sauer reagiert. Das ausgefallene Säureadditionssalz wird in geeigneter Weise, zum Beispiel durch Filtrieren, abgetrennt.

Die erfindungsgemäßen Verbindungen der allgemeinen Formel I und ihre Säureadditionssalze können gewünschtenfalls einer weiteren Reinigung, zum Beispiel durch Umkristallisieren, unterzogen werden. Das zum Umkristallisieren eingesetzte Lösungsmittel wird in Abhängigkeit von der Löslichkeit und den Kristallisationseigenschaften des zu kristallisierenden Stoffes gewählt.

Ferner sind erfindungsgemäß Arzneimittel, welche 1 oder mehr erfindungsgemäße Verbindung(en) als Wirkstoff(e), zweckmäßigerweise zusammen mit 1 oder mehr üblichen pharmazeutischen Träger-, Streck- und/oder Hilfs- beziehungsweise Zusatzstoff(en), enthalten, vorgesehen. Die erfindungsgemäßen

- 12 -

BAD ORIGINAL

1-[2'-(substituierte)-5',6',7',8'-Tetra-(hydro)-4'-(oxo)-chinazolin-6'-yl]-3,4-di-[hydro]-6,7-di-[substituierte]-isochinolinderivate haben nämlich wertvolle pharmakologische, insbesondere krampflösende, schmerz- und fiebersenkende und bei akuter Alkoholvergiftung schützende Wirkungen.

Die Wirkstoffe der allgemeinen Formel I und ihre physiologisch verträglichen Säureadditionssalze können mit den in der Arzneimittelherstellung üblichen, zur parenteralen oder enteralen Applikation geeigneten, nichttoxischen festen und/oder flüssigen Trägerstoffen und/oder Hilfsstoffen vermischt und zu Arzneimittelpräparaten formuliert werden. Als Trägerstoffe finden zum Beispiel Wasser, Gelatine, Lactose, Stärke, Pektin, Magnesiumstearat, Stearinsäure, Talk, Pflanzenöle (wie Erdnußöl und/oder Olivenöl) Verwendung. Die Wirkstoffe werden zu den üblichen Darreichungsformen formuliert, zum Beispiel festen Präparaten (abgerundeten oder eckigen Tabletten, Dragees, Kapseln, z.B. Hartgelatinekapseln, Pillen, Zäpfchen) oder flüssigen Präparaten (mit Öl oder Wasser bereiteten Lösungen, Suspensionen, Emulsionen, Sirupen, Weichgelatinekapseln, mit Öl oder Wasser bereiteten injizierbaren Lösungen oder Suspensionen). Die Menge des festen Trägerstoffes kann in einem weiten Bereich variieren, eine Darreichungseinheit enthält vorzugsweise etwa 25 mg bis 1 g Trägerstoff. Die Präparate können gegebenenfalls die üblichen Hilfsstoffe, zum Beispiel Konservierungs-, Stabilisierungs-, Netz- und Emulgiermittel, Salze zum Einstellen des osmotischen Druckes, Puffer, Geruchs- und Geschmacksstoffe, enthalten. Die Präparate können weitere pharmazeutisch wertvolle, bekannte Verbindungen enthalten, ohne daß dadurch eine synergistische Wirkung einträte. Die Präparate werden vorzugsweise in Darreichungseinheiten formuliert, die der gewünschten Applikationsform entsprechen. Die Arzneimittelpräparate werden mittels der dafür üblichen



BAD ORIGINAL

Verfahren hergestellt, zum Beispiel durch Sieben, Mischen, Granulieren und Pressen der Komponenten oder durch Auflösen. Die Präparate können ferner weiteren, in der Arzneimittelindustrie üblichen Arbeitsgängen unterzogen, zum Beispiel sterilisiert werden.

Für die im folgenden geschilderten pharmakologischen Tests wurden CFLP-Mäuse (LATI) beiderlei Geschlechts und mit einem Gewicht von 18-22 g, ferner Han. Wistar-Ratten mit einem Gewicht von 160-180 g verwendet. Die zu testenden Substanzen wurden peroral in Dosen von 30 mg/kg in Form einer 5 % Tween® 80 enthaltenden Suspension eine Stunde vor Versuchsbeginn verabreicht.

## Pharmakologische Untersuchungen

### Testmethoden

#### 1. Metrazolkrampf (MET) (an Mäusen)

Nach der Vorbehandlung wurden den Tieren 125 mg/kg Pentylentetrazol subkutan appliziert. Als geschützt wurden diejenigen Tiere betrachtet, die überlebten und bei denen der tonische Extensorkrampfanfall unterblieb. Die Beobachtungsdauer betrug eine Stunde (Everett, L.M. und Richards, R.K.: J. Pharmacol. Exp. Ther. 81 402 /1944/).

#### 2. Strychninkrampf (STRY) (an Mäusen)

Nach einer Stunde Vorbehandlung wurde der tonische Extensorkrampfanfall mit 2,5 mg/kg intraperitoneal appliziertem Strychninnitrat ausgelöst. Als geschützt wurden diejenigen Tiere betrachtet, bei denen wegen der Behandlung der Krampfanfall unterblieb (Kerley, T.L. et al.: J. Pharmacol. Exp. Ther. 132, 360 /1961/).



BAD ORIGINAL

3. Schmerzstillende Wirkung (FCS) (an Mäusen)

Eine Stunde nach der Vorbehandlung wurde den Tieren als Schmerzreiz 0,6 %ige Essigsäure intraperitoneal injiziert (0,3 ml). Die Häufigkeit des Writhing-Syndroms wurde 30 Minuten lang registriert. Die durch die Behandlung auftretende Veränderung wurde zu den Durchschnittswerten der Kontrolle in Beziehung gesetzt und der Unterschied in Prozent ausgedrückt (Koster, R. et al., J. Pharmacol. Exp. Ther. 72, 74 /1941/).

4. Fiebersenkende Wirkung (LCS) (an Ratten)

Bei Ratten wurden mit einer Bierhefesuspension (0,5 % Bierhefe, 1 %iges Gummi arabicum, 0,3 ml s.c.) Hyperthermie hervorgerufen. Vier Stunden später wurden die Tiere mit den zu untersuchenden Stoffen behandelt, und die tracheale Temperatur der Tiere wurde stündlich, 4 Stunden lang mit einem ELAB-Thermometer (Typ. TE-3) registriert. Die fiebersenkende Wirkung wurde durch Prozente der Tiere ausgedrückt, bei denen die Temperatur 1 °C niedriger als der Durchschnitt der Temperatur der mit dem Lösungsmittel behandelten Kontrollgruppe ist (Nimegeers, C.J.E. et al.: Arzneim. Forsch. 25, 1591 /1975/).

5. Akute Alkoholvergiftung (ETA) (an Ratten)

Eine Stunde nach der Behandlung wurden die Tiere mit Äthylalkohol in einer Dosis von 3,5 g/kg intraperitoneal behandelt. Die Schlafzeit der Tiere wurde registriert, die Durchschnittsschlafdauer der einzelnen Gruppen wurde mit der Schlafzeit der Kontrolle verglichen und der Unterschied in Prozent ausgedrückt.

Die Ergebnisse sind in der nachstehenden Tabelle I angegeben.



BAD ORIGINAL

0161478

Die untersuchten Verbindungen sind wie folgt:

"A" = 1-[2'-(Piperidin-1''-yl)-5',6',7',8'-tetra-(hydro)-4'-(oxo)-chinazolin-6'-yl]-3,4-di-[hydro]-6,7-di-[methoxy]-isochinolin.

"B" = 1-[2'-(4''-{p-⟨Chlor⟩-phenyl}-4''-{hydroxy}-piperidin-1''-yl)-5',6' 7',8'-tetra-(hydro)-4'-(oxo)-chinazolin-6'-yl]-3,4-di-[hydro]-6,7-di-[methoxy]-isochinolin.

"C" = 1-[2'-(4''-{Piperidin-1'''-yl}-4''-{carbamoyl}-piperidin-1''-yl)-5',6',7',8'-tetra-(hydro)-4'-(oxo)-chinazolin-6'-yl]-3,4-di-[hydro]-6,7-di-[methoxy]-isochinolin.

"D" = 1-[2'-(3'',4'',5''-Tri-{methoxy}-phenyl)-5',6',7' 8'-tetra-(hydro)-4'-(oxo)-chinazolin-6'-yl]-3,4-di-[hydro]-6,7-di-[methoxy]-isochinolin.

"E" = 1-[2'-(1'',2'',5'',6''-Tetra{hydro}-4''-{phenyl}-pyrid-1''-yl)-5',6',7',8'-tetra-(hydro)-4'-(oxo)-chinazolin-6'-yl]-3,4-di-[hydro]-6,7-di-[methoxy]-isochinolin.

"F" = 1-[2'-(4''-{2'''-⟨Chlor⟩-phenyl}-piperazin-1''-yl)-5',6',7',8'-tetra-(hydro)-4'-(oxo)-chinazolin-6'-yl]-3,4-di-[hydro]-6,7-di-[methoxy]-isochinolin.

"G" = 1-[2'-(3''-⟨Methyl⟩-phenyl)-5',6',7',8'-tetra-(hydro)-4'-(oxo)-chinazolin-6'-yl]-3,4-di-[hydro]-6,7-di-[methoxy]-isochinolin.

"H" = 1-[2'-(Pyrid-3''-yl)-5',6',7',8'-tetra-(hydro)-4'-(oxo)-chinazolin-6'-yl]-3,4-di-[hydro]-6,7-di-[methoxy]-isochinolin.

- 16 -

BAD ORIGINAL

"I" = 1-[2'-(pyrrolidin-1''-yl)-5',6',7',8'-tetra--(hydro)-4'-(oxo)-chinazolin-6'-yl]-3,4-di-[hydro]--6,7-di-[methoxy]-isochinolin.

Tabelle I

| Verbindung | MET | STRY | FCS | LCS | ETA |
|---|---|---|---|---|---|
| "A" | | | -40 % | | |
| "B" | | | | -40 % | -45 % |
| "C" | | | | | -58 % |
| "D" | 40 | | | | |
| "E" | | 40 | | | |
| "F" | 40 | | | | |
| "G" $ED_{50}$ = 21,2 mg/kg | | | | | |
| "H" | | | | | -40 % |
| "I" | 40 | 40 | $ED_{50}$=19,3 mg/kg | -40 % | |

Aus den Tabellenangaben ist ersichtlich, daß insbesondere die Verbindung "G" eine bedeutende krampflösende Wirkung aufweist. Ihr $ED_{50}$-Wert beträgt 21,2 mg/kg. Vom Gesichtspunkt der schmerzstillenden Wirkung erwies sich die Verbindung "I" als am wirksamsten mit einem $ED_{50}$-Wert von 19,3 mg/kg. Bei den Verbindungen "B" und "I" kann auch eine bedeutende fiebersenkende Wirkung beobachtet werden, während die Verbindungen "B", "C" und "H" bei akuten Alkoholvergiftungen schützend wirken.

Die Erfindung wird an Hand der folgenden Beispiele näher erläutert.



BAD ORIGINAL

<u>Beispiele 1 bis 18</u>

1-[2'-(Phenyl)-5',6',7',8'-tetra-(hydro)--4'-(oxo)-chinazolin-6'-yl]-3,4-di-[hydro]--6,7-di-[methoxy]-isochinolin

16,7 mg (0,05 Mol) 1-[3'-(Methoxycarbonyl)-4'-(oxo)--cyclohex-1'-yl]-3,4-di-[hydro]-6,7-di-[methoxy]-isochinolin und 8,6 g (0,055 Mol) Benzamidin-hydrochlorid werden in 200 ml wasserfreiem Äthylalkohol suspendiert und die Lösung von 1,26 g (0,055 Grammatom) Natriummetall und 50 ml wasserfreiem Äthanol wird zugesetzt. Das Reaktionsgemisch wird 6 Stunden lang unter Rühren und Rückfluß gekocht und das Lösungsmittel abdestilliert. Der Rückstand wird in 100 ml Wasser suspendiert, durch Vakuumfiltern isoliert, mit Wasser und dann mit Aceton gewaschen und getrocknet. Aus Toluol kristallisiert wird er gereinigt.

Die physikalischen und analytischen Angaben des erhaltenen Produktes [Beispiel 1] und von aus 1-[3'--(Methoxycarbonyl)- beziehungsweise 1-[3'-(Äthoxycarbonyl)--4'-(oxo)-cyclohex-1'-yl]-3,4-di-[hydro]-6,7-di-[methoxy]- beziehungsweise -6,7-di-[äthoxy]-isochinolin auf ähnliche Weise hergestellten weiteren 2'-(substituierten) Derivaten [Beispiele 2 bis 18] sind in der Tabelle II enthalten.

Die salzsauren Salze der hergestellten Verbindungen werden so hergestellt, daß die Base in wäßrigem Alkohol suspendiert und durch das Zusetzen einer anderthalbfachen Menge wäßriger Salzsäure aufgelöst, eingedampft und mit wasserfreiem oder 96 %igem Äthanol verrieben filtriert und dann umkristallisiert wird.



BAD ORIGINAL

Tabelle II

1-[2'-$R_3$-(substituierte)-5',6',7',8'-Tetra-(hydro)-4'-(oxo)-chinazolin-6'-yl]-3,4-di-[hydro]-6,7-di-[methoxy]- beziehungsweise -6,7-di-[äthoxy]-isochinoline der allgemeinen Formel I

| Beispiel Nr. | $R_1 = R_2$ | $R_3$ | Salz HX | Summenformel Molgewicht | Schmp. °C Lösungsmittel | | Analyse, % berechnet | gefunden | Ausbeute (%) |
|---|---|---|---|---|---|---|---|---|---|
| 1 | $-OCH_3$ | $-C_6H_5$ | - | $C_{25}H_{27}O_3N_3$ | 248 | C | 71,92 | 72,13 | |
| | | | | | | H | 6,51 | 6,38 | 66 |
| | | | | 417,51 | Toluol | N | 10,0 | 9,98 | |
| 2 | $-OCH_3$ | $-C_6H_5$ | HCl | $C_{25}H_{28}O_3N_3Cl$ | 275-276 | C | 66,43 | 66,22 | |
| | | | | | 90 %iges | H | 5,79 | 6,08 | |
| | | | | 453,97 | Äthanol | N | 9,29 | 9,12 | |
| 3 | $-OCH_3$ | $-C_6H_4Cl(m)$ | - | $C_{25}H_{24}N_3O_3Cl$ | 154-155 | C | 66,73 | 66,30 | |
| | | | | | | H | 5,57 | 5,68 | 72 |
| | | | | 449,94 | DMF | N | 9,33 | 9,07 | |
| 4 | $-OCH_3$ | $-C_6H_4Cl(m)$ | HCl | $C_{25}H_{25}N_3O_3Cl_2$ | 288-289 | C | 61,73 | 62,02 | |
| | | | | | 96 %iges | H | 5,18 | 5,35 | |
| | | | | 486,40 | Äthanol | N | 8,63 | 8,51 | |



BAD ORIGINAL

**Tabelle II**
(Fortsetzung)

| Bei-spiel Nr. | $R_1 = R_2$ | $R_3$ | Salz HX | Summenformel Molgewicht | Schmp. °C Lösungsmittel | | Analyse, % berechnet | gefunden | Ausbeute (%) |
|---|---|---|---|---|---|---|---|---|---|
| 5 | $-OCH_3$ | $-C_6H_3(OCH_3)_2$(m.p) | - | $C_{27}H_{29}N_3O_5$ | 275 | C | 68,19 | 67,86 | |
| | | | | | | H | 6,14 | 6,25 | 70 |
| | | | | 475,54 | Toluol | N | 8,89 | 8,88 | |
| 6 | $-OCH_3$ | $-C_6H_3(OCH_3)_2$(m.p) | HCl | $C_{27}H_{30}N_3O_5Cl$ | 252-253 | C | 63,37 | 63,70 | |
| | | | | | 96 %iges | H | 5,40 | 6,12 | |
| | | | | 512,01 | Äthanol | N | 8,20 | 8,35 | |
| 7 | $-OCH_3$ | $-CH_3$ | - | $C_{20}H_{23}N_3O_3$ | 260-261 | C | 67,97 | 67,85 | |
| | | | | | 96 %iges | H | 6,55 | 6,72 | 52 |
| | | | | 353,42 | Äthanol | N | 11,88 | 11,56 | |
| 8 | $-OCH_3$ | $-CH_3$ | 2HCl | $C_{20}H_{25}N_3O_3Cl_2$ | 268-269 | C | 56,34 | 56,58 | |
| | | | | | 96 %iges | H | 5,91 | 6,16 | |
| | | | | 426,34 | Äthanol | N | 9,85 | 10,2 | |
| 9 | $-OCH_3$ | $-CH_2C_6H_4Cl$(p) | - | $C_{26}H_{26}N_3O_3Cl$ | 286-287 | C | 67,30 | 67,56 | |
| | | | | | | H | 5,64 | 5,90 | 78 |
| | | | | 463,96 | DMF | N | 9,05 | 9,09 | |

- 20 -

BAD ORIGINAL

Tabelle II
(Fortsetzung)

| Beispiel Nr. | $R_1 = R_2$ | $R_3$ | Salz HX | Summenformel Molgewicht | Schmp. °C Lösungsmittel | | Analyse, % berechnet | gefunden | Ausbeute (%) |
|---|---|---|---|---|---|---|---|---|---|
| 10 | $-OCH_3$ | $-CH_2C_6H_4Cl$(p) | 2HCl | $C_{26}H_{28}N_3O_3Cl_3$ | 249-250 | C | 58,16 | 58,18 | |
| | | | | | 96 %iges | H | 5,69 | 5,35 | |
| | | | | 536,89 | Äthanol-Äther | N | 7,82 | 7,47 | |
| 11 | $-OCH_3$ | Pyrid-4-yl | - | $C_{24}H_{24}N_4O_3$ | 258-259 | C | 69,21 | 68,86 | 54 |
| | | | | | | H | 5,80 | 6,17 | |
| | | | | 416,48 | Toluol | N | 13,45 | 13,28 | |
| 12 | $-OCH_3$ | Pyrid-4-yl | 2HCl | $C_{24}H_{26}N_4O_3Cl_2$ | 265-266 | C | 58,90 | 58,73 | |
| | | | | | 96 %iges | H | 5,35 | 5,75 | |
| | | | | 489,40 | Äthanol-Äther | N | 11,44 | 11,80 | |
| 13 | $-OC_2H_5$ | $-C_6H_4Cl$(m) | - | $C_{27}H_{28}N_3O_3Cl$ | 198 | C | 67,84 | 68,22 | |
| | | | | | Äthanol | H | 5,90 | 6,19 | 55 |
| | | | | 477,99 | (unter Zersetzung) | N | 8,79 | 8,37 | |
| 14 | $-OC_2H_5$ | $-C_6H_4Cl$(m) | HCl | $C_{27}H_{29}N_3O_3Cl_2$ | | C | 63,03 | 63,28 | |
| | | | | | Äthanol | H | 5,68 | 6,04 | |
| | | | | | | N | 8,16 | 7,59 | |
| | | | | 514,45 | | Cl | 13,78 | 14,04 | |



BAD ORIGINAL

Tabelle II
(Fortsetzung)

| Beispiel Nr. | $R_1 = R_2$ | $R_3$ | Salz HX | Summenformel Molgewicht | Schmp. °C Lösungsmittel | | Analyse, % berechnet | gefunden | Ausbeute (%) |
|---|---|---|---|---|---|---|---|---|---|
| 15 | $-OC_2H_5$ | $-CH_3$ | - | $C_{22}H_{27}N_3O_3$ | 225 | C | 69,26 | 68,57 | |
| | | | | | (unter | H | 7,13 | 7,15 | 48 |
| | | | | 381,47 | Zersetzung) | N | 11,01 | 10,80 | |
| | | | | | Äthanol | | | | |
| 16 | $-OC_2H_5$ | $-CH_3$ | HBr | $C_{22}H_{28}N_3O_3Br$ | 255 | C | 57,14 | 56,77 | |
| | | | | | (unter | H | 6,10 | 6,24 | |
| | | | | 462,39 | Zersetzung) | N | 9,08 | 8,97 | |
| | | | | | Äthanol | | | | |
| 17 | $-OC_2H_5$ | $-CH_2C_6H_4Cl(p)$ | - | $C_{28}H_{30}N_3O_3Cl$ | 250 | C | 68,35 | 68,35 | |
| | | | | | (unter | H | 6,14 | 6,83 | 58 |
| | | | | 492,02 | Zersetzung) | N | 8,54 | 8,56 | |
| | | | | | DMF | | | | |
| 18 | $-OC_2H_5$ | $-CH_2C_6H_4Cl(p)$ | HBr | $C_{28}H_{31}N_3O_3BrCl$ | 261 | C | 58,69 | 58,34 | |
| | | | | | (unter | H | 5,45 | 5,65 | |
| | | | | 572,93 | Zersetzung) | N | 7,33 | 7,12 | |
| | | | | | Äthanol | | | | |



BAD ORIGINAL

0161478

## Beispiele 19 bis 40

1-[2'-(Piperidin-1''-yl)-5',6',7',8'-tetra-(hydro)-4'-(oxo)-chinazolin-6'-yl]-3,4-di-[hydro]-6,7-di-[methoxy]-isochinolin

16,7 g (0,05 Mol) 1-[3'-(Methoxycarbonyl)-4'-(oxo)-cyclohex-1'-yl]-3,4-di-[hydro]-6,7-di-[methoxy]-isochinolin und 9,69 g (0,0275 Mol) Piperidino-guanidin-sulfat werden in 200 ml wasserfreiem Äthanol suspendiert, und eine Lösung von 1,26 g (0,055 Grammatom) Natriummetall und 50 ml wasserfreiem Alkohol wird zugesetzt. Das Reaktionsgemisch wird 6 Stunden lang unter Rühren und Rückfluß gekocht und das Lösungsmittel abdestilliert. Der Rückstand wird in 100 ml Wasser suspendiert, durch Vakuumfiltern abgetrennt, mit Wasser und dann mit Aceton gewaschen und getrocknet. Aus Toluol oder Äthanol kristallisiert wird er gereinigt.

Die physikalischen und analytischen Daten des erhaltenen Produktes [Beispiel 19] und von aus 1-[3'-(Methoxycarbonyl)- beziehungsweise 1-[3'-(Äthoxycarbonyl)-4'-(oxo)-cyclohex-1'-yl]-3,4-di-[hydro]-6,7-di-[methoxy]-beziehungsweise -6,7-di-[äthoxy]-isochinolin auf ähnliche Weise hergestellten 2'-(substituierten) Derivaten [Beispiele 20 bis 40] sind in Tabelle III enthalten.

Die salzsauren Salze der hergestellten Verbindungen werden wie in den Beispielen 1 bis 18 beschrieben hergestellt.



BAD ORIGINAL

Tabelle III

1-[2'-$R_3$-(substituierte)-5',6',7' 8'-Tetra-(hydro)-4'-(oxo)-chinazolin-6'-yl]-3,4-di-[hydro]-6,7-di-[methoxy]-isochinoline der allgemeinen Formel I

| Beispiel Nr. | $R_1$ = $R_2$ | $R_3$ | Salz HX | Summenformel Molgewicht | Schmp. °C Lösungsmittel | | Analyse, % berechnet | gefunden | Ausbeute (%) |
|---|---|---|---|---|---|---|---|---|---|
| 19 | $-OCH_3$ | Piperidino | - | $C_{24}H_{30}N_4O_3$ | 278-79 | C | 68,22 | 68,33 | |
| | | | | | | H | 7,15 | 7,37 | 64 |
| | | | | 422,53 | Äthanol | N | 13,25 | 13,48 | |
| 20 | $-OCH_3$ | Piperidino | 2 HCl | $C_{24}H_{32}N_4O_3Cl_2$ | 248-250 | C | 58,18 | 57,86 | |
| | | | | | (unter | H | 6,51 | 6,29 | |
| | | | | | Zersetzung) | N | 11,30 | 10,95 | |
| | | | | 495,44 | Äthanol-Äther | Cl | 14,31 | 14,66 | |
| 21 | $-OCH_3$ | Morpholin-4-yl | - | $C_{23}H_{28}N_4O_4$ | 286 | C | 65,07 | 65,45 | |
| | | | | | (unter | H | 6,64 | 6,52 | 52 |
| | | | | 424,50 | Zersetzung) | N | 13,19 | 13,49 | |
| | | | | | Äthanol | | | | |
| 22 | $-OCH_3$ | Morpholin-4-yl | 2 HCl | $C_{23}H_{30}N_4O_4Cl_2$ | 234-235 | C | 55,55 | 55,24 | |
| | | | | | (unter | H | 6,08 | 6,41 | |
| | | | | | Zersetzung) | N | 11,26 | 10,95 | |
| | | | | 497,42 | Äthanol-Äther | Cl | 14,22 | 14,60 | |



0161478

BAD ORIGINAL

Tabelle III
(Fortsetzung)

| Beispiel Nr. | $R_1 = R_2$ | $R_3$ | Salz HX | Summenformel Molgewicht | Schmp. °C Lösungsmittel | | Analyse, % berechnet | gefunden | Ausbeute (%) |
|---|---|---|---|---|---|---|---|---|---|
| 23 | $-OCH_3$ | 1-Methyl-piperazin-4-yl | 3 HCl | $C_{24}H_{31}N_5O_3$ | 241 | C | 65,88 | 66,17 | |
| | | | | | | H | 7,14 | 7,50 | 58 |
| | | | | 437,54 | Äthanol | N | 16,00 | 15,72 | |
| 24 | $-OCH_3$ | 1-Methyl-piperazin--4-yl | 3 HCl | $C_{24}H_{34}N_5O_3Cl_3$ | 258-260 | C | 52,70 | 52,31 | |
| | | | | | (unter | H | 6,26 | 6,54 | |
| | | | | | Zersetzung) | N | 12,80 | 12,40 | |
| | | | | 546,92 | Äthanol-Äther | Cl | 19,44 | 19,10 | |
| 25 | $-OCH_3$ | 1-Phenyl-piperazin--4-yl | - | $C_{29}H_{33}N_5O_3$ | 241-242 | C | 69,71 | 70,08 | |
| | | | | | | H | 6,65 | 6,82 | 65 |
| | | | | 499,62 | DMF | N | 14,01 | 13,89 | |
| 26 | $-OCH_3$ | 1-Phenyl-piperazin--4-yl | 3 HCl | $C_{29}H_{36}N_5O_3Cl_3$ | 232-234 | C | 57,19 | 56,85 | |
| | | | | | | H | 5,95 | 6,30 | |
| | | | | 609,00 | Äthanol | N | 11,49 | 11,98 | |
| 27 | $-OCH_3$ | 4-Piperidino-4-carbamoyl-piperidin-1-yl | 3 HCl | $C_{30}H_{43}N_6O_4Cl_3$ | 243-245 | C | 54,75 | 54,43 | |
| | | | | | (unter | H | 6,58 | 6,97 | 47 |
| | | | | 658,07 | Zersetzung) | N | 12,77 | 12,80 | |
| | | | | | Äthanol | Cl | 16,16 | 15,82 | |



BAD ORIGINAL

Tabelle III
(Fortsetzung)

| Beispiel Nr. | $R_1 = R_2$ | $R_3$ | Salz HX | Summenformel Molgewicht | Schmp. °C Lösungsmittel | | Analyse, % berechnet | gefunden | Ausbeute (%) |
|---|---|---|---|---|---|---|---|---|---|
| 28 | $-OCH_3$ | 4-Phenyl-4-hydroxy--piperidin-1-yl | - | $C_{30}H_{34}N_4O_4Cl$ | 160-162 | C | 65,50 | 65,87 | |
| | | | | | | H | 6,23 | 6,55 | 61 |
| | | | | 550,08 | DMF | N | 10,18 | 9,88 | |
| 29 | $-OCH_3$ | 4-Phenyl-4-hydroxy--piperidin-1-yl | 2 HCl | $C_{30}H_{36}N_4O_4Cl_3$ | über 350 | C | 57,83 | 57,45 | |
| | | | | | (ab 270 | H | 5,92 | 6,13 | |
| | | | | 623,60 | Zersetzung) | N | 8,99 | 8,63 | |
| | | | | | Wasser | Cl | 17,07 | 17,40 | |
| 30 | $-OCH_3$ | 4-Phenyl-1,2,5,6--tetrahydro-piridin-1-yl | - | $C_{30}H_{32}O_3N_4$ | 217-218 | C | 72,55 | 72,82 | |
| | | | | | | H | 6,49 | 6,70 | 64 |
| | | | | 496,61 | Toluol | N | 11,28 | 11,44 | |
| 31 | $-OCH_3$ | 4-Phenyl-1,2,5,6--tetrahydro-piridin-1--yl | 2 HCl | $C_{30}H_{34}O_3N_4Cl_2$ | 250-255 | C | 63,26 | 63,28 | |
| | | | | | (unter | H | 6,01 | 6,37 | |
| | | | | 569,53 | Zersetzung) | N | 9,83 | 9,80 | |
| | | | | | 96 %iges Äthanol | | | | |
| 32 | $-OCH_3$ | 2-Trifluormethyl--benzyl | - | $C_{27}H_{26}N_3O_3F_3$ | 269-270 | C | 67,52 | 68,49 | |
| | | | | | | H | 7,33 | 7,41 | 57 |
| | | | | 497,52 | DMF | N | 9,26 | 10,11 | |



BAD ORIGINAL

Tabelle III
(Fortsetzung)

| Beispiel Nr. | $R_1 = R_2$ | $R_3$ | Salz HX | Summenformel Molgewicht | Schmp. °C Lösungsmittel | | Analyse, % berechnet | gefunden | Ausbeute (%) |
|---|---|---|---|---|---|---|---|---|---|
| 33 | $-OCH_3$ | Pyrrolidino | - | $C_{23}H_{28}N_4O_3$ | 274-275 | C | 67,67 | 67,85 | |
| | | | | | | H | 6,9 | 7,2 | 62 |
| | | | | 408,60 | Äthanol | N | 13,71 | 13,55 | |
| 34 | $-OCH_3$ | Pyrid-3-yl | - | $C_{24}H_{24}N_4O_3$ | 221-222 | C | 69,21 | 69,41 | |
| | | | | | | H | 5,80 | 6,12 | 56 |
| | | | | 416,48 | Äthanol | N | 13,45 | 13,22 | |
| 35 | $-OCH_3$ | 3-Methyl-phenyl | - | $C_{26}H_{27}N_3O_3$ | 234-235 | C | 72,70 | 73,11 | |
| | | | | | | H | 6,33 | 6,68 | 58 |
| | | | | 429,52 | Äthanol | N | 9,78 | 10,05 | |
| 36 | $-OCH_3$ | 4-[2'-Cl-Phenyl]-piperazin-1-yl | - | $C_{29}H_{32}N_5O_3Cl$ | 254 | C | 65,22 | 64,95 | |
| | | | | | (unter | H | 6,03 | 6,27 | 57 |
| | | | | 534,06 | Zersetzung) DMF | N | 13,11 | 13,03 | |
| 37 | $-OCH_3$ | 3,4,5-Trimethoxy-phenyl | 2HCl | $C_{28}H_{33}N_3O_6Cl_2$ | 218-220 | C | 62,04 | 61,70 | |
| | | | | | | H | 5,95 | 5,74 | 61 |
| | | | | 578,49 | Äthanol-Äther | N | 12,25 | 11,96 | |



BAD ORIGINAL

Tabelle III
(Fortsetzung)

| Bei-spiel Nr. | $R_1 = R_2$ | $R_3$ | Salz HX | Summenformel Molgewicht | Schmp. °C Lösungs-mittel | | Analyse, % berechnet | gefunden | Ausbeute (%) |
|---|---|---|---|---|---|---|---|---|---|
| 38 | $-OCH_3$ | N-Methyl-piperazino | 3HCl | $C_{24}H_{34}N_5O_3Cl_3$ | 258-260 | C | 52,70 | 52,31 | |
| | | | | | (unter | H | 6,26 | 6,54 | 53 |
| | | | | 546,92 | Zersetzung) | N | 12,80 | 12,40 | |
| | | | | | Äthanol-Äther | Cl | 19,44 | 19,10 | |
| 39 | $CH_3O-$ | 4-(4'-Cl-Phenyl)-4-OH--piperidino | 2HCl | $C_{30}H_{36}N_4O_4Cl_3$ | ab 270 | C | 57,83 | 57,45 | |
| | | | | | (Zersetzung) | H | 5,82 | 6,11 | 51 |
| | | | | 623,00 | Äthanol-Äther | N | 8,99 | 8,63 | |
| | | | | | | Cl | 17,07 | 17,40 | |
| 40 | $CH_3O-$ | Piperidino | 2HCl | $C_{24}H_{32}N_4O_3Cl_2$ | 248-250 | C | 58,98 | 57,86 | |
| | | | | | Zersetzung) | H | 6,51 | 6,29 | 56 |
| | | | | 495,44 | Äthanol-Äther | N | 11,30 | 10,95 | |



BAD ORIGINAL

Beispiel 41

1-[2'-(Phenyl)-5',6',7',8'-tetra-(hydro)-
-4'-(oxo)-chinazolin-6'-yl]-3,4-di-[hydro]-
-6,7-di-[hydroxy]-isochinolin-hydrobromid

8,34 g (0,02 Mol) 1-[(2'-(Phenyl)-5',6',7',8'-tetra-(hydro)-4'-(oxo)-chinazolin-6'-yl]-3,4-di-[hydro]-6,7-di-[methoxy]-isochinolin werden in 40 ml 40 %iger wäßriger Bromwasserstoffsäure suspendiert und am Ölbad eine Stunde lang schwach gekocht. Durch das Erhitzen löst sich der Ausgangsstoff langsam - im Verhältnis zum Reaktionsablauf - auf. Der Reaktionsablauf wird durch Dünnschichtchromatographie kontrolliert. Nach Beendigung der Umsetzung wird im Vakuum bis zur Trockne eingedampft und dann aus wäßrigem Äthanol kristallisiert.

Ausbeute: 78 %.
Schmelzpunkt: 293-295 °C

Elementaranalyse für die Formel $C_{23}H_{23}N_3O_3Br$ (549,26):
berechnet: C % = 50,29, H % = 4,22, N % = 7,65;
gefunden: C % = 50,61, H % = 4,55, N % = 7,29.

Patentansprüche

0161478

## Patentansprüche

1.) 1-[2'-(substituierte)-5',6',7',8'-Tetra-(hydro)--4'-(oxo)-chinazolin-6'-yl]-3,4-di-[hydro]-6,7-di--[substituierte]-isochinolinderivate der allgemeinen Formel

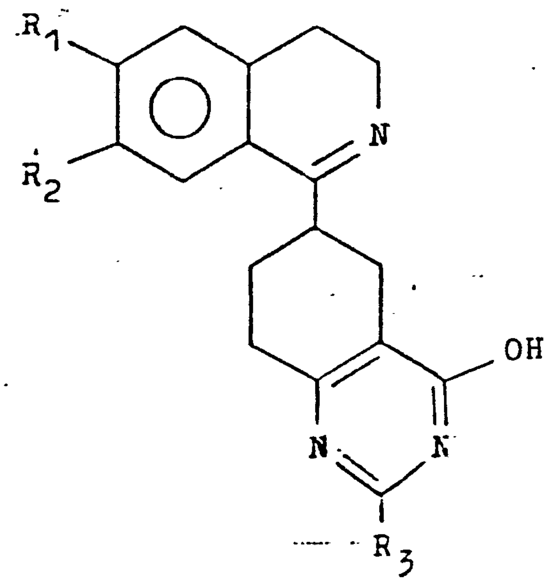

beziehungsweise I,

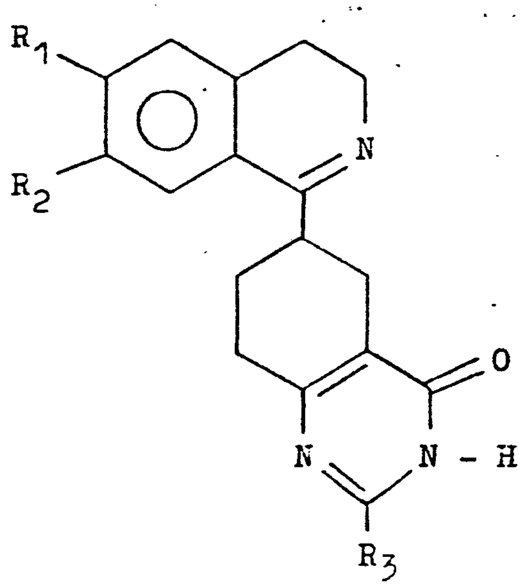

worin

$R_1$ und $R_2$ unabhängig voneinander für Hydroxygruppen beziehungsweise Alkoxyreste mit 1 bis 6 Kohlenstoffatom(en) stehen und

$R_3$ einen Alkylrest mit 1 bis 6 Kohlenstoffatom(en), einen, gegebenenfalls substituierten, Phenylrest, einen, gegebenenfalls substituierten, Alkylphenylrest mit 1 bis 4 Kohlenstoffatom(en) im Alkylteil oder einen, gegebenenfalls substituierten, heterocyclischen Rest bedeutet,

sowie ihre Säureadditionssalze und quaternären Salze.

2.) 1-[2'-(substituierte)-5',6',7',8'-Tetra-(hydro)--4'-(oxo)-chinazolin-6'-yl]-3,4-di-[hydro]-6,7-di--[substituierte]-isochinolinderivate nach Anspruch 1, dadurch gekennzeichnet, daß der beziehungsweise die Alkoxyrest(e) und/oder Alkylrest(e), für welche[n] $R_1$ und/oder $R_2$ und/oder $R_3$ stehen kann beziehungsweise können, [ein] solche[r] mit 1 bis 4, insbesondere 1 oder 2, Kohlenstoffatom(en) ist beziehungsweise sind.

3.) 1-[2'-(substituierte)-5',6',7',8'-Tetra-(hydro)--4'-(oxo)-chinazolin-6'-yl]-3,4-di-[hydro]-6,7-di--[substituierte]-isochinolinderivate nach Anspruch 1 oder 2, dadurch gekennzeichnet, daß der Alkylphenylrest, für welchen $R_3$ stehen kann, ein solcher mit 1 oder 2 Kohlenstoffatom(en) im Alkylteil ist.

4.) 1-[2'-(substituierte)-5',6',7',8'-Tetra-(hydro)--4'-(oxo)-chinazolin-6'-yl]-3,4-di-[hydro]-6,7-di--[substituierte]-isochinolinderivate nach Anspruch 1 bis 3, dadurch gekennzeichnet, daß der beziehungsweise die Substituent(en), durch welche[n] der Phenylrest oder Alkylphenylrest, für den $R_3$ stehen kann, substituiert sein kann, 1 oder mehr Halogenatom(e), insbesondere Chloratom(e), Alkoxyrest(e) mit 1 bis 6, insbesondere 1 bis 4, Kohlenstoffatom(en), Alkylrest(e) mit 1 bis 6, insbesondere 1 bis 4, Kohlenstoffatom(en) und/oder Trihalogenmethylrest(e), insbesondere Trifluormethylrest(e), ist beziehungsweise sind.

5.) 1-[2'-(substituierte)-5',6',7',8'-Tetra-(hydro)--4'-(oxo)-chinazolin-6'-yl]-3,4-di-[hydro]-6,7-di--[substituierte]-isochinolinderivate nach Anspruch 1 oder 2, dadurch gekennzeichnet, daß der heterocyclische Rest, für den $R_3$ stehen kann, 5 bis 7-gliedrig, insbesondere 5- oder 6-gliedrig ist und als Heteroatom(e) 1 oder mehr Stickstoff-, Sauerstoff- und/oder Schwefelatom(e) aufweist, vor allem ein Pyridyl-, Piperidinyl-, Morpholinyl-, Piperazinyl-, 1,2,5,6-Tetrahydropyridyl- oder Pyrrolidinylrest, ist.

6.) 1-[2'-(substituierte)-5',6',7',8'-Tetra-(hydro)--4'-(oxo)-chinazolin-6'-yl]-3,4-di-[hydro]-6,7-di--[substituierte]-isochinolinderivate nach Anspruch 1, 2 oder 5, dadurch gekennzeichnet, daß der beziehungsweise die Substituent(en), durch welche[n] der heterocyclische Rest, für den $R_3$ stehen kann, substituiert sein kann, 1 oder mehr Alkylrest(e) mit 1 bis 4, insbesondere 1 oder 2, Kohlenstoffatom(en), gegebenenfalls durch 1 oder mehr Halogenatom(e), insbesondere Chloratom(e), substituierte[r], Phenylrest(e), Hydroxygruppe(n), Carbamoylrest(e) und/oder weitere[r], vorteilhaft 5- bis 7-gliedrige[r], insbesondere 5- oder 6-gliedrige[r], und als Heteroatom(e) 1 oder mehr Stickstoff-, Sauerstoff- und/oder Schwefelatom(e) aufweisende[r], heterocyclische[r] Rest(e), vor allem [ein] Pyridyl-, Piperidinyl-, Morpholinyl-, Piperazinyl-, 1,2,5,6-Tetrahydropyridyl- oder Pyrrolidinylrest, ist beziehungsweise sind.

7.) 1-[2'-(Piperidin-1''-yl)-5',6',7',8'-tetra-(hydro)-4'-(oxo)-chinazolin-6'-yl]-3,4-di-[hydro]-6,7-di-[methoxy]-isochinolin, 1-[2'-(4''-{p-⟨Chlor⟩-phenyl}-4''-{hydroxy}-piperidin-1''-yl)-5',6',7' 8'-tetra-(hydro)-4'-(oxo)-chinazolin-6'-yl]-3,4-di-[hydro]-6,7-di-[methoxy]-isochinolin, 1-[2'-(4''-{Piperidin-1'''-yl}-4''-{carbamoyl}-piperidin-1''-yl)-5',6',7',8'-tetra-(hydro)-4'-(oxo)-chinazolin-6'-yl]-3,4-di-[hydro]-6,7-di-[methoxy]-isochinolin, 1-[2'-(3'',4'',5''-Tri-{methoxy}-phenyl)-5',6',7',8'-tetra-(hydro)-4'-(oxo)-chinazolin-6'-yl]-3,4-di-[hydro]-6,7-di-[methoxy]-isochinolin, 1-[2'-(1'',2'',5'',6''-Tetra-{hydro}-4''-{phenyl}-pyrid-1''-yl)-5',6',7',8'-tetra-(hydro)-4'-(oxo)-chinazolin-6'-yl]-3,4-di-[hydro]-6,7-di-[methoxy]-isochinolin, 1-[2'-(4''-{2'''-⟨Chlor⟩-phenyl}-piperazin-1''-yl)-5',6',7',8'-tetra-(hydro)-4'-(oxo)-chinazolin-6'-yl]-3,4-di-[hydro]-6,7-di-[methoxy]-isochinolin, 1-[2'-(3''-⟨Methyl⟩-phenyl)-5',6',7',8'-tetra-(hydro)-4'-(oxo)-chinazolin-6'-yl]-3,4-di-[hydro]-6,7-di-[methoxy]-isochinolin, 1-[2'-(Pyrid-3''-yl)-5',6',7',8'-tetra-(hydro)-4'-(oxo)-chinazolin-6'-yl]-3,4-di-[hydro]-6,7-di-[methoxy]-isochinolin und 1-[2'-

-(Pyrrolidin-1''-yl)-5',6',7',8'-tetra--(hydro)-4'-(oxo)-chinazolin-6'-yl]-3,4-di-[hydro]--6,7-di-[methoxy]-isochinolin sowie ihre Säureadditionssalze und quaternären Salze.

8.) Verfahren zur Herstellung der Verbindungen nach Anspruch 1 bis 7, dadurch gekennzeichnet, daß man 1-[3'-(Carboxy)-4'-(oxo)-cyclohex-1'-yl]-3,4-di--[hydro]-6,7-di-[substituierte]-isochinolinderivate beziehungsweise 1-[3'-(Carboxy)-4'-(imino)-cyclohex-1'-yl]-3,4-di-[hydro]-6,7-di-[substituierte]--isochinolinderivate der allgemeinen Formel

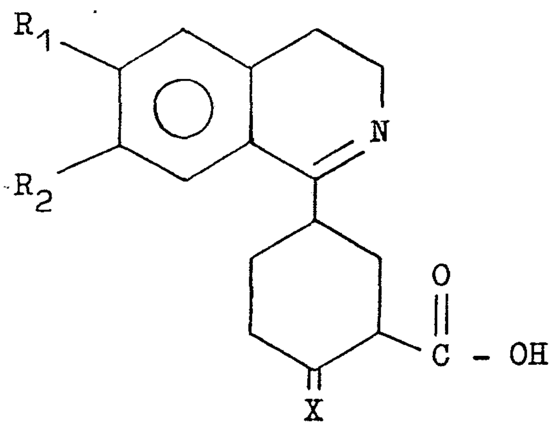

II ,

worin

| | |
|---|---|
| $R_1$ und $R_2$ | die im Anspruch 1 oder 2 angegebenen Bedeutungen haben und |
| X | für ein Sauerstoffatom oder eine Iminogruppe steht, |

oder reaktionsfähige Derivate derselben mit Amidinen der allgemeinen Formel

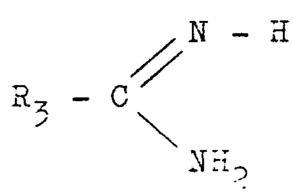

III ,



BAD ORIGINAL

worin $R_3$ die in den Ansprüchen 1 oder 3 bis 6 angegebenen Bedeutungen hat, oder mit Salzen derselben mit anorganischen oder organischen Säuren in einem basischen Medium kondensiert,

worauf man

gegebenenfalls in den erhaltenen 1-[2'-(substituierte)-5',6',7',8'-Tetra-(hydro)-4'-(oxo)-chinazolin-6'-yl]-3,4-di-[hydro]-6,7-di-[substituierte]-isochinolinderivaten der allgemeinen Formel I den beziehungsweise die Substituenten, für welche[n] $R_1$ und/oder $R_2$ und/oder $R_3$ steht beziehungsweise stehen, zu [einem] anderen Substituenten, welche[n] $R_1$ und/oder $R_2$ und/oder $R_3$ bedeuten kann beziehungsweise können, umsetzt

und/oder

gegebenenfalls die erhaltenen 1-[2'-(substituierte)-5',6',7',8'-Tetra-(hydro)-4'-(oxo)-chinazolin-6'-yl]-3,4-di-[hydro]-6,7-di-[substituierte]-isochinolinderivate der allgemeinen Formel I in Säureadditionssalze oder quaternäre Salze überführt und/oder gegebenenfalls die erhaltenen Säureadditionssalze der 1-[2'-(substituierte)- 5',6',7',8'-Tetra-(hydro)-4'-(oxo)-chinazolin-6'-yl]-3,4-di-[hydro]-6,7-di-[substituierte]-isochinolinderivate der allgemeinen Formel I in die freien 1-[2'-(substituierte)-5',6',7',8'-Tetra-(hydro)-4'-(oxo)-chinazolin-6'-yl]-3,4-di-[hydro]-6,7-di-[substituierte]-isochinolinderivate der allgemeinen Formel I oder in andere Säureadditionssalze oder quaternäre Salze überführt.

9.) Verfahren nach Anspruch 8, dadurch gekennzeichnet, daß man zur Herstellung des basischen Mediums Alkalimetallhydroxyde, -carbonate und/oder -alkoholate verwendet.

10.) Arzneimittel, gekennzeichnet durch einen Gehalt an 1 oder mehr Verbindung(en) nach Anspruch 1 bis 7 als Wirkstoff(en), zweckmäßigerweise zusammen mit 1 oder mehr üblichen pharmazeutischen Träger-, Streck- und/oder Hilfs- beziehungsweise Zusatzstoff(en).

Zusammenfassung

BAD ORIGINAL